# EUROPEAN PATENT APPLICATION

(11) **EP 3 401 677 A1**
(43) Date of publication of application: **14.11.2018**
(21) Application number: 16883839.9
(22) Date of filing: 26.12.2016
(51) Int. Cl.: G01N 33/48, G01N 21/64, G01N 33/483

(54) **LIVING BODY OBSERVATION METHOD AND LIVING BODY OBSERVATION DEVICE**

(30) Priority: 05.01.2016 JP 2016000422
(71) Applicant: Hamamatsu Photonics K.K., Hamamatsu-shi, Shizuoka 435-8558 (JP)
(72) Inventor: MIWA Mitsuharu, Hamamatsu-shi Shizuoka 435-8558 (JP)
(74) Representative: Hoffmann Eitle
(86) International application number: PCT/JP2016/088708
(87) International publication number: WO 2017/119340

(57) **Abstract**

A living body observation device is a device that allows observation of a surface of a specimen, and includes an excitation light source that radiates excitation light including a wavelength between 300 nm and 650 nm, a filter that cuts the excitation light in observation light emitted from a specimen irradiated with the excitation light, a camera that captures the observation light and outputs image data, a data storage unit that stores an image of the observation light as a reference image, an image processing unit that acquires a target image that is an image of the observation light after acquisition of the reference image and generates an analysis image indicating change in light intensity of the target image with respect to the reference image, and a display that outputs the analysis image.

## Description

### Technical Field

One aspect of the present invention relates to a living body observation method and a living body observation device for observing an observation target.

### Technical Field

In the related art, a technology for identifying cancer cells in a tissue excised from a living body using a reagent has been developed. For example, in Non-Patent Document 1 below, it has been clarified that a mammary gland tumor can be selectively caused to be brighter and a surrounding mammary gland and a mammary gland tumor in adipose tissue can be identified by spraying a fluorescent reagent of which the fluorescence changes due to a specific protein breakdown enzyme of which the activity is increased in cancer cells onto a specimen removed in mammary gland surgery.

### Citation List

### Non Patent Literature

[Non-Patent Document 1] "Rapid intraoperative visualization of breast lesions with γ-glutamyl hydroxymethyl rhodamine green", Scientific reports, July 13, 2015

### Summary of Invention

### Technical Problem

In the scheme described in Non-Patent Document 1, it is seen that fluorescence is generated from adipose tissue or the like other than cancer cells in the tissue. Therefore, when a fluorescent image is observed from tissue excised from a living body after a fluorescent reagent is applied to the tissue, it is desired to facilitate determination of the presence or absence or the position of cancer cells in the tissue.

Therefore, the present invention has been made in view of such a problem, and an object of the present invention is to provide a living body observation method and a living body observation device capable of accurately determining the presence or absence or a position of cancer cells on a surface of a tissue.

### Solution to Problem

In order to solve the above problem, a living body observation method according to one embodiment of the present invention is a living body observation method for observing a surface of an observation target excised from a living body, and includes: applying a fluorescent substance that emits fluorescence by binding to a cancer cell and has an excitation wavelength in a range of 300 nm to 650 nm to the observation target; irradiating the observation target with excitation light including the excitation wavelength using a light source; cutting the excitation light from observation light emitted from the observation target using a filter; capturing the observation light from which the excitation light has been cut and outputting image data using a camera; storing a reference image which is an image of the observation light after the fluorescent substance is applied on the basis of the image data; acquiring a target image which is an image of the observation light after acquisition of the reference image on the basis of the image data and generating an analysis image indicating change in light intensity of the target image with respect to the reference image using an image processing device; and outputting the analysis image using an output device.

According to the living body observation method of the above aspect, after the fluorescent substance that emits fluorescence by binding to a cancer cell is applied to the observation target excised from the living body, the observation light emitted from the observation target according to irradiation with excitation light is captured in a state in which the excitation light has been cut. On the basis of the image data obtained as a result, the image of the observation light after the fluorescent substance is applied is obtained as the reference image, the image of the observation light after the acquisition of the reference image is obtained as the target image, and then, the analysis image indicating change in light intensity of the target image with respect to the reference image is generated and output. Accordingly, it is possible to accurately determine the presence or absence or a position of cancer cells in a tissue on the basis of change in the light intensity of the target image with respect to the reference image.

Alternatively, a living body observation device according to another embodiment of the present invention is a living body observation device that allows observation of a surface of an observation target, and includes: a light source configured to radiate excitation light including a wavelength between 300 nm and 650 nm; a filter configured to cut the excitation light in observation light emitted from the observation target irradiated with the excitation light; an imaging unit configured to capture the observation light from which the excitation light has been cut, and output image data; a storage unit configured to store a reference image that is an image of the observation light emitted from the observation target on the basis of the image data; an image processing device configured to acquire a target image that is an image of the observation light after acquisition of the reference image on the basis of the image data and generate an analysis image indicating change in light intensity of the target image with respect to the reference image; and an output device configured to output the analysis image.

According to the living body observation device of the above aspect, the observation light emitted from the observation target according to the irradiation with the excitation light is captured in a state in which the excitation light has been cut. If there are cancer cells on a surface of the observation target, the observation light includes fluorescence caused by the cancer cells. On the basis of the image data obtained as a result, the image of the observation light is obtained as the reference image, the image of the observation light after the acquisition of the reference image is obtained as the target image, and then, the analysis image indicating change in light intensity of the target image with respect to the reference image is generated and output. Accordingly, it is possible to accurately determine the presence or absence or a position of cancer cells in a tissue on the basis of change in the light intensity of the target image with respect to the reference image.

### Advantageous Effects of Invention

According to an aspect of the present invention, it is possible to accurately determine the presence or absence or a position of cancer cells on a surface of a tissue.

### Brief Description of Drawings

FIG. 1 is a diagram illustrating a schematic configuration of a living body observation device 1 according to a preferred embodiment of the present invention.
FIG. 2 is a block diagram illustrating a configuration of a computer 15 in FIG. 1 including an internal functional configuration.
FIG. 3 is a flowchart illustrating a procedure of an analysis image generation process in the living body observation device 1 of FIG. 1.
FIG. 4 is a diagram illustrating a schematic configuration of a living body observation device 1A according to a modification example of the present invention.
FIG. 5 is a diagram illustrating a schematic configuration of a living body observation device IB according to another modification example of the present invention.
FIG. 6 is a diagram illustrating a schematic configuration of a living body observation device 1C according to still another modification example of the present invention.
FIG. 7 is a diagram illustrating a configuration of an experimental system including a simulation device.
FIG. 8 is a diagram illustrating a disposition state of a sample that is a test target.
FIG. 9 is a diagram illustrating an analysis image of experimental results obtained by the experimental system of FIG. 7.

### Description of Embodiments

Hereinafter, preferred embodiments of a living body observation device and a living body observation method according to the present invention will be described in detail with reference to the drawings. In the description of the drawings, the same or corresponding parts are denoted by the same reference numerals, and duplicated description thereof will be omitted.

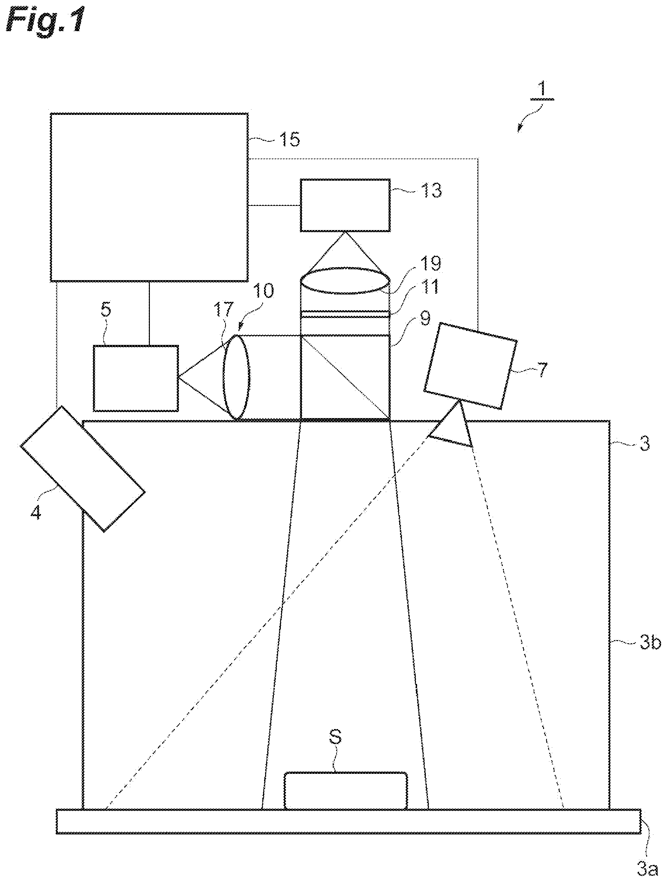

FIG. 1 is a block diagram illustrating a schematic configuration of a living body observation device 1 according to a preferred embodiment of the present invention. The living body observation device 1 illustrated in FIG. 1 is a device for observing a surface of a specimen S that is an observation target. The specimen S is, for example, a sample excised from a living body such as a human being, an animal, or the like. The living body observation device 1 includes a dark box 3 that accommodates the specimen S, an illumination light source 4, an excitation light source 5, a spraying device 7, a dichroic mirror 9, an excitation light shielding filter 11, a camera (imaging unit) 13, and a computer 15. Note that the excitation light source 5, the dichroic mirror 9, and the excitation light shielding filter 11 constitute an excitation light irradiation unit 10.

The dark box 3 is configured of a bottom plate 3a on which the specimen S is placed, and a light-shielding cover 3b, and is a member for preventing interfering light from being incident on the specimen S. The illumination light source 4 outputs illumination light with which the specimen S is irradiated. For example, the illumination light source 4 may be a light source device that irradiates the specimen S in the dark box 3 with illumination light, and be electrically connected to the computer 15. Further, the excitation light source 5 may be a light source device for irradiating the specimen S with excitation light via an optical system including the dichroic mirror 9 and the like, and be electrically connected to the computer 15. A wavelength of the excitation light radiated by the excitation light source 5 is set to a wavelength between 300 nm and 650 nm. Specifically, a collimator lens 17 is disposed between the excitation light source 5 and the dichroic mirror 9, and the excitation light radiated from the excitation light source 5 is changed into parallel light by the collimator lens 17, reflected by the dichroic mirror 9, and then, radiated to the specimen S in the dark box 3. Turning the light irradiation by the illumination light source 4 and the excitation light source 5 on and off is controlled by an instruction signal of the computer 15. The dichroic mirror 9 is attached to an upper surface of the cover 3b of the dark box 3, transmits the fluorescence emitted from the specimen S in the dark box 3 and the illumination light reflected from the surface of the specimen S toward the camera 13, and reflects the excitation light radiated from the excitation light source 5 toward the specimen S in the dark box 3. That is, the dichroic mirror 9 serves to separate an optical path of the excitation light from the excitation light source 5 from an optical path of the illumination light and the fluorescence from the specimen S.

In order to prevent the illumination light output from the illumination light source 4 and reflected by the surface of the specimen S from being shielded by the dichroic mirror 9, a wavelength of the illumination light may be set as a fluorescence wavelength. Further, in order to capture a reflection image of the specimen S, a manual or automatic slide mechanism for disposing or removing the dichroic mirror 9 and the excitation light shielding filter 11 with respect to the specimen S and the optical axis of the camera 13 may be provided. In this case, the automatic slide mechanism may be controlled in conjunction with the control of the illumination light source 4 and the excitation light source 5 so that the dichroic mirror 9 and the excitation light shielding filter 11 are removed from the optical axis when the illumination light source 4 is ON, and the dichroic mirror 9 and the excitation light shielding filter 11 are disposed on the optical axis when the excitation light source 5 is ON.

The camera 13 is a device that captures, as observation light, the fluorescence emitted from the specimen S irradiated with the excitation light and the illumination light reflected from the surface of the specimen S irradiated with the illumination light, and is fixed above the cover 3b with the dichroic mirror 9 interposed therebetween outside the dark box 3. The camera 13 is electrically connected to the computer 15, and outputs moving image data or still image data showing images of the fluorescence and the illumination light to the computer 15. As the camera 13 having such a function, an area image sensor such as a CCD area image sensor or a CMOS area image sensor is used. Further, an imaging lens 19 that focuses the observation light transmitted through the dichroic mirror 9 onto the camera 13, and an excitation light shielding filter 11 that cuts the excitation light from the observation light including the fluorescence from the specimen S transmitted through the dichroic mirror 9 are disposed between the camera 13 and the dichroic mirror 9. Note that the excitation light shielding filter 11 may be omitted when the dichroic mirror 9 has a function of completely cutting the excitation light.

The spraying device 7 has a mechanism that accommodates a fluorescent substance therein and sprays the fluorescent substance toward the specimen S in the dark box 3. The spraying device 7 is electrically connected to the computer 15 and sprays the fluorescent substance according to an instruction signal from the computer 15. A fluorescent reagent that emits fluorescence when the fluorescent reagent binds to a cancer cell and of which an excitation peak wavelength (excitation wavelength) is within the range of 300 nm to 650 nm is used as the fluorescent substance accommodated in the spraying device 7. In particular, the excitation wavelength is preferably in the range of 300 nm to 600 nm. Since light absorption by hemoglobin is high in such a wavelength range, it is difficult for the excitation light to reach the inside of the specimen S. Therefore, the surface of the specimen S can be suitably observed. For example, a fluorescent reagent of which an excitation peak wavelength is 496 nm, and a fluorescence peak wavelength is 525 nm and that reacts with a cancer cell having a γ-glutamyltranspeptidase (GGT) activity and emits fluorescence can be used as an example of a fluorescent reagent having an excitation wavelength in such a wavelength range.

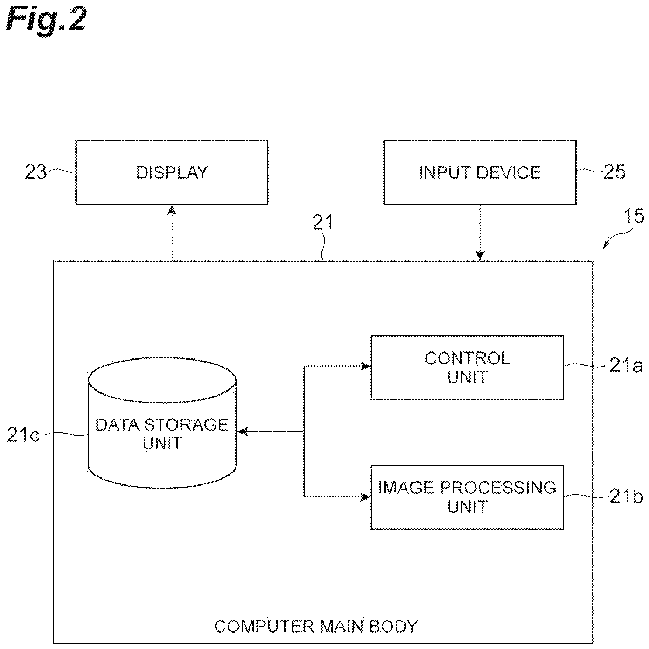

The computer 15 is an information processing device including a built-in memory such as a RAM and a ROM and a processor (an arithmetic circuit) such as a CPU, and controls an operation of each unit of the living body observation device 1 and processes image data output from the camera 13. FIG. 2 illustrates a configuration of the computer 15 including an internal functional configuration. The computer 15 includes a computer main body 21, and a display 23 and an input device 25 electrically connected to the computer main body 21. The input device 25 is configured of a touch panel, a keyboard, a mouse, and the like, receives instruction content input from a user, and transfers the instruction content to the computer main body 21. The display 23 is an output device that displays (outputs) data of processing results of the computer main body 21.

A control unit 21a, an image processing unit 21b operating as an image processing device, and a data storage unit 21c are mounted as functional components on the computer main body 21. The control unit 21a controls operations of the camera 13, the illumination light source 4, the excitation light source 5, and the spraying device 7. Specifically, the control unit 21a controls a shutter timing (an exposure timing) which is an capturing timing of the camera 13. Further, the control unit 21a controls a light emission timing of the illumination light source 4 and the excitation light source 5. Further, the control unit 21a controls a spraying timing of the fluorescent substance in the spraying device 7. The data storage unit 21c stores the image data output from the camera 13. The image processing unit 21b executes image processing on the image data stored in the data storage unit 21c to generate an analysis image and displays the analysis image on the display 23.

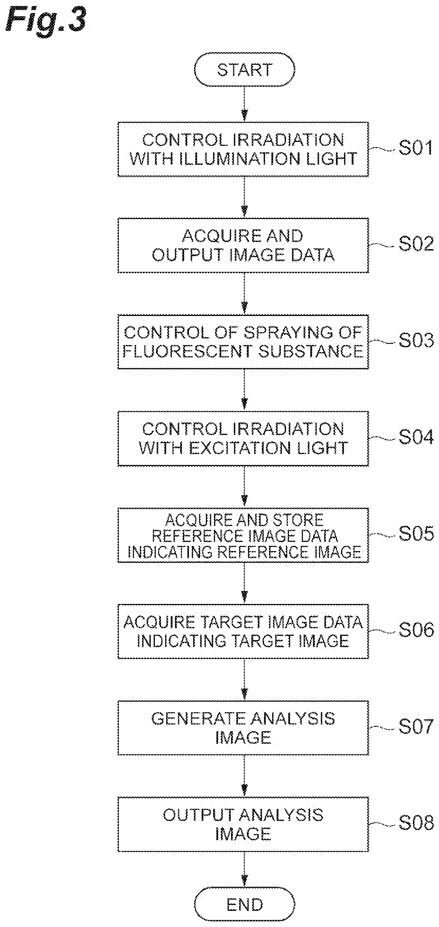

Hereinafter, a procedure of the analysis image generation process in the above-described living body observation device 1 will be described, and the living body observation method according to the embodiment will be described in detail. FIG. 3 is a flowchart illustrating a procedure of an analysis image generation process in the living body observation device 1.

First, imaging processing of the specimen S is started in a state in which the specimen S is placed on the bottom plate 3a of the dark box 3 according to an instruction input by the user to the computer 15. Accordingly, the illumination light source 4 is controlled by the computer 15 such that illumination light is radiated from the illumination light source 4 toward the surface of the specimen S (step S01). In synchronization with this, the computer 15 performs control such that the reflected light reflected by the surface of the specimen S is captured by the camera 13. As a result, the image data output from the camera 13 is acquired by the computer 15, and the image data is displayed on the display 23 (step S02). Thus, by displaying the image of the specimen S illuminated with the illumination light on the display 23, the user can adjust the disposition of the specimen S while viewing the display image. Note that since the fluorescent substance has not been applied to the specimen S in this state, fluorescence is not generated from the specimen S. Therefore, the image data output from the camera 13 does not include a fluorescent image. Note that each time the image data output from the camera 13 is received, the image data is stored in the data storage unit 21c of the computer 15.

Thereafter, the spraying device 7 is controlled by the computer 15 according to an instruction input from the user in a state in which the specimen S is placed on the bottom plate 3a of the dark box 3, such that a predetermined amount of the fluorescent substance is sprayed toward the surface of the specimen S (step S03). Accordingly, a fluorescent substance is applied to the surface of the specimen S. Information on a spraying timing of the fluorescent substance is stored in the data storage unit 21c by the control unit 21a of the computer 15.

Further, the excitation light source 5 is controlled by the computer 15 immediately after the spraying of the fluorescent substance, such that radiation of the excitation light including a wavelength for exciting the fluorescent substance directed to the specimen S is started (step S04). Accordingly, the excitation light radiated from the excitation light source 5 is reflected by the dichroic mirror 9 and is incident on the surface of the specimen S. Accordingly, fluorescence is generated from the surface of the specimen S to which the fluorescent substance is applied. The observation light including the fluorescence emitted from the specimen S is captured by the camera 13 via the dichroic mirror 9 and the excitation light shielding filter 11. Accordingly, the excitation light reflected and scattered by the specimen S is cut by the dichroic mirror 9 and the excitation light shielding filter 11. In this case, it is preferable for the timing of irradiation of the excitation light by the excitation light source 5 to be controlled such that the timing is synchronized with the capturing timing of the camera 13. Further, the irradiation with the excitation light and the capturing by the camera 13 are controlled such that the irradiation and the imaging continue until at least three minutes have elapsed after the fluorescent substance is sprayed.

In the specimen S to which the fluorescent substance is applied, fluorescence may also be emitted from other tissues such as fat as well as cancer cells. However, since the fluorescent substance has a property that it changes into a fluorescence due to a specific protein breakdown enzyme of which the activity is increased in the cancer cells, the intensity of the fluorescence caused by the cancer cells tends to increase with the elapse of time in the specimen S to which such a fluorescent substance has been applied. In order to use such properties, an analysis image is obtained as follows in the embodiment.

That is, in the computer 15, reference image data indicating a reference image which is the image of the observation light immediately after the fluorescent substance is sprayed is acquired on the basis of the image data output from the camera 13 and the information on the spraying timing of the fluorescent substance stored in the data storage unit 21c, and the reference image data is stored in the data storage unit 21c (step S05). Further, the reference image data may be acquired as the image of the observation light after a predetermined initial time (for example, 30 seconds) has elapsed after the fluorescent substance is sprayed.

Further, target image data indicating a target image which is the image of the observation light after a predetermined time longer than an initial period has elapsed after the fluorescent substance is sprayed is acquired on the basis of the image data output from the camera 13, and the target image data is stored in the data storage unit 21c (step S06). The predetermined time for determining a timing of capturing the target image is set to at least three minutes or more and, preferably, to three minutes or more and less than five minutes.

Then, the image processing unit 21b of the computer 15 acquires the reference image data and the target image data from the data storage unit 21c, and generates a difference image of these as analysis image data (step S07). The analysis image data generated in this way indicates a distribution on the surface of the specimen S of change in light intensity of the target image with respect to the reference image. Finally, the image processing unit 21b of the computer 15 outputs the analysis image indicated by the analysis image data to the display 23 (step S08). This analysis image data may be superimposed on the image data of the reflection image of the specimen S acquired in step S02 and displayed, and may be displayed as a three-dimensional image using a plurality of pieces of analysis image data acquired by capturing being performed at various angles by the camera 13.

According to the living body observation device 1 and the living body observation method using the living body observation device 1 described above, a fluorescent substance that emits fluorescence by reacting with cancer cells is applied to the specimen S excised from the living body, and then, the observation light emitted from the specimen S according to irradiation with excitation light is captured in a state in which the excitation light is cut. On the basis of the image data obtained as a result, the image of the observation light immediately after the fluorescent substance is applied is obtained as the reference image, the image of the observation light after a predetermined time has elapsed from the acquisition of the reference image is obtained as the target image, and then, the analysis image indicating change in light intensity of the target image with respect to the reference image is generated and output. In the specimen to which the fluorescent substance is applied, there is little temporal change in the fluorescence generated from tissues such as fat, but the intensity of the fluorescence from the cancer cells changes (increases) with time. It is considered that it is possible to distinguish cancer cells from other tissues in the specimen by using such properties. In the embodiment, it is possible to accurately determine the position of cancer cells in the tissue on the basis of change in light intensity of the target image with respect to the reference image. Further, the fluorescent substance used in the embodiment has an excitation wavelength in a wavelength region which is easily absorbed by hemoglobin. Therefore, when there is a part of which the fluorescence intensity has changed in the specimen, it is considered that there is a high likelihood of there being cancer cells on the surface of the specimen. Accordingly, according to the embodiment, it is possible to easily determine whether or not there are cancer cells on the surface of the specimen.

In the embodiment, the difference between the reference image and the target image is generated as the analysis image. With such a configuration, it is possible to clearly visualize a portion in which the change in the light intensity of the target image with respect to the reference image is large. Accordingly, the accuracy of determination of a position of cancer cells in the tissue can be improved.

Further, in the embodiment, the reference image data indicating the reference image output from the camera 13 is stored in the data storage unit 21c of the computer 15. In this case, it is possible to easily acquire change in the light intensity of the target image with respect to the reference image. As a result, accuracy in determining the position of cancer cells in the tissue can be ensured.

Further, in the embodiment, the image of the observation light immediately after the fluorescent substance is applied or the image of the observation light after the initial period has elapsed after the fluorescent substance is applied is stored as the reference image. In this case, it is possible to easily acquire change in the light intensity of the target image with respect to the reference image. As a result, it is possible to ensure accuracy in determination of the presence or absence or the position of cancer cells on the tissue surface.

Further, in the embodiment, the image of the observation light after at least three minutes have elapsed after the fluorescent substance is applied is acquired as the target image. It is assumed that temporal change in fluorescence from the cancer cells becomes sufficiently large after three minutes have elapsed after the fluorescent substance is applied. Therefore, it is possible to clearly visualize the change in the light intensity of the target image with respect to the reference image using the target image acquired after at least three minutes has elapsed after the fluorescent substance is applied. Accordingly, the accuracy of determination of the position of cancer cells in the tissue can be improved. On the other hand, it is also assumed that temporal change in fluorescence from the cancer cells becomes small after five minutes or more has elapsed from the application of the fluorescent substance. Therefore, change in the light intensity of the target image with respect to the reference image can be more clearly visualized using the target image acquired after a predetermined time of three minutes or more and less than five minutes has elapsed after the fluorescent substance is applied.

Note that the present invention is not limited to the above-described embodiment.

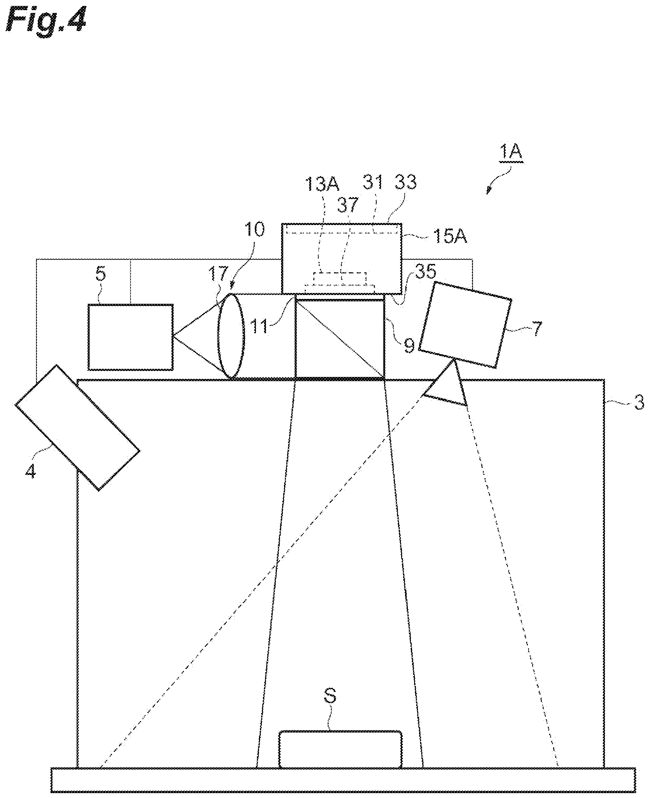

For example, the configuration of the living body observation device 1 according to the above-described embodiment may be changed to the configuration illustrated in FIGS. 4, 5, and 6.

That is, as in the living body observation device 1A according to the modification example illustrated in FIG. 4, the computer 15 and the camera 13 of the living body observation device 1 may be replaced with a smart device 15A included a camera (imaging unit) 13A built thereinto. In this living body observation device 1A, since a lens is built into the camera 13A, the imaging lens 19 is omitted. The smart device 15A is a terminal device such as a smartphone or a tablet terminal, and a configuration of the smart device 15A is the same as that of the computer 15 illustrated in FIG. 2, except that the camera 13A is built in. Specifically, a control unit 21a, an image processing unit 21b, and a data storage unit 21c are mounted on the smart device 15A as functional components. The excitation light source 5 is electrically connected to the smart device 15A, and the control unit 21a has a function of controlling the excitation light source 5 so that the excitation light source 5 radiates the excitation light in synchronization with the capturing timing of the camera 13A.

The smart device 15A includes a main surface 33 having a display 31 and a back surface 35 opposite to the main surface 33, which is a back surface 35 on which the excitation light irradiation unit 10 is disposed. An opening 37 (first opening) for guiding the observation light emitted from the specimen S to the camera 13A is provided on the back surface 35.

With the configuration including the smart device 15A, capturing, storage of image data, and transfer of the image data are facilitated. As a result, it is possible to efficiently generate the analysis image indicating change in light intensity of the target image with respect to the reference image. Further, the entire device configuration can be easily simplified.

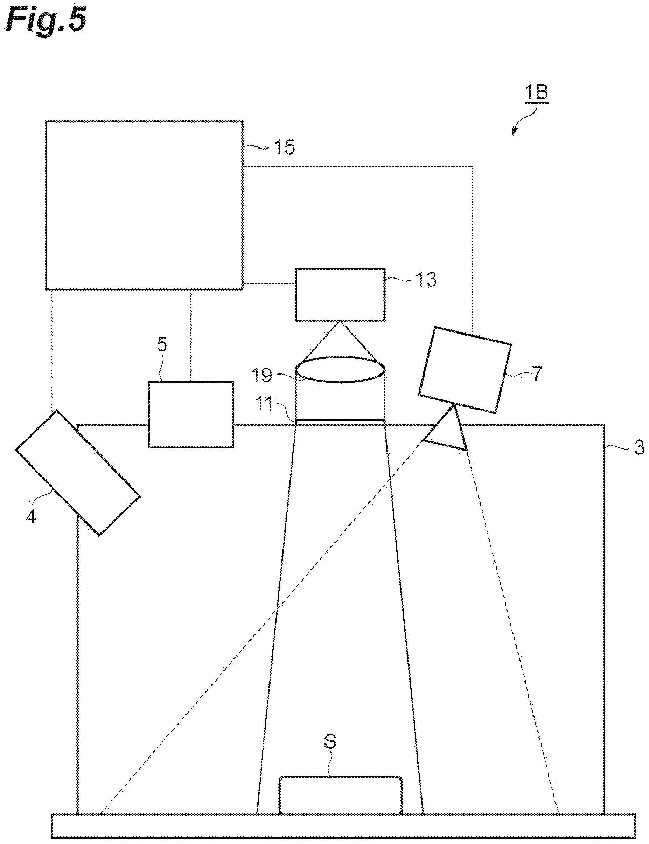

Further, the dichroic mirror 9 and the collimator lens 17 of the living body observation device 1 may be omitted, as in the living body observation device IB according to the modification example illustrated in FIG. 5. In the living body observation device IB having such a configuration, the excitation light source 5 is configured to be able to directly irradiate the specimen S in the dark box 3 with the excitation light. Further, the camera 13 is configured to capture the observation light from the specimen S via the excitation light shielding filter 11 and the imaging lens 19.

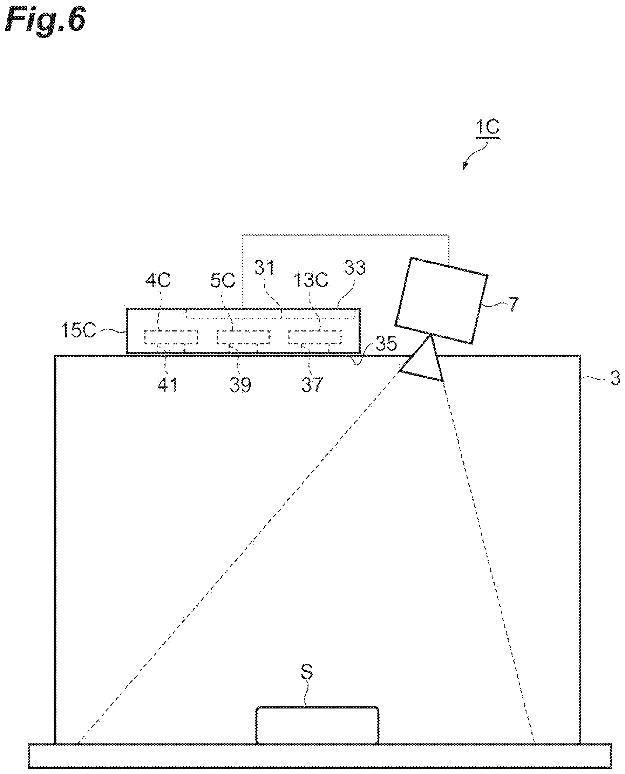

Further, the computer 15 and the camera 13 of the living body observation device 1, the illumination light source 4, and the excitation light source 5 may be replaced with a smart device 15C including a camera (imaging unit) 13C, an illumination light source 4C, and an excitation light source 5C, as in the living body observation device 1C according to the modification example illustrated in FIG. 6. In this living body observation device 1C, the collimator lens 17 and the dichroic mirror 9 are omitted. The smart device 15C has the same configuration as the computer 15 illustrated in FIG. 2 except that the camera 13C, the illumination light source 4 C, and the excitation light source 5 C are built into the smart device 15C. Specifically, the control unit 21a, the image processing unit 21b, and the data storage unit 21c are mounted on in the smart device 15C as functional components. The control unit 21a of the smart device 15C has a function of controlling the excitation light source 5C so that the excitation light source 5C radiates the excitation light in synchronization with the capturing timing of the camera 13C.

Further, the smart device 15C includes a main surface 33 having a display 31, and a back surface 35 that is a surface opposite to the main surface 33. The smart device 15C includes, on the back surface 35, an opening 37 (a first opening) for guiding the observation light emitted from the specimen S to the camera 13C, an opening 39 (a second opening) for guiding the excitation light output from the excitation light source 5C, and an opening 41 (a third opening) for guiding the illumination light output from the illumination light source 4C. An excitation light shielding filter is disposed inside the opening 37.

In the living body observation devices 1, 1A, IB, and 1C, the image processing unit 21b is not limited to the generation of a difference image between the reference image data and the target image data as analysis image data. For example, a division image (an image indicating an intensity ratio) between the reference image data and the target image data may be generated as the analysis image data. Further, the image processing unit 21b may generate, as the analysis image data, an image obtained by performing image processing such as a binarization process on an analysis image such as a difference image or a division image.

Further, the observation target is not limited to a specimen excised from a living body such as a human being or an animal, and a tongue or skin of a human being, an animal, or the like, an incision site due to a surgical operation, or the like may be the observation target. In this case, the fluorescent substance may be applied to the tongue, the skin, the incision site, or the like by spraying, application, or the like, or autofluorescence emitted from the observation target may be captured as observation light without applying a fluorescent substance. An excitation wavelength of autofluorescence is in a range of 300 nm to 450 nm. In such a wavelength region, since the light absorption of hemoglobin is large, it is difficult for the excitation light to reach the inside of the observation target, and the surface of the observation target can be suitably observed. When autofluorescence is observed, the image processing unit 21b may create a fluorescence image of the autofluorescence.

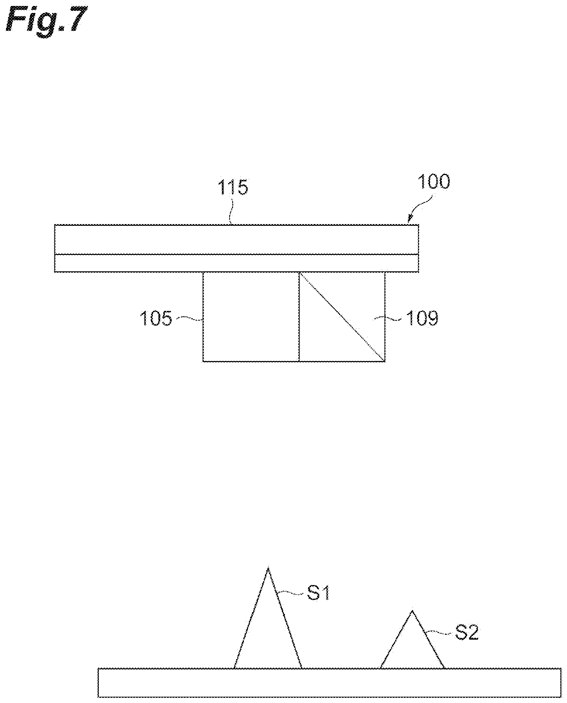

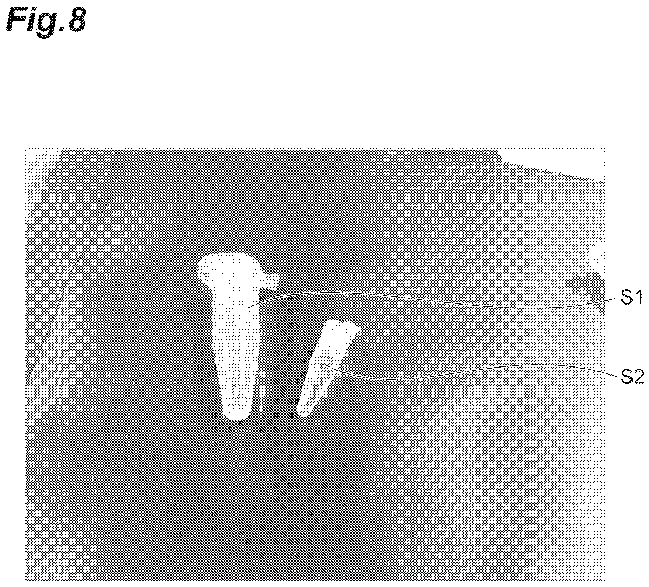

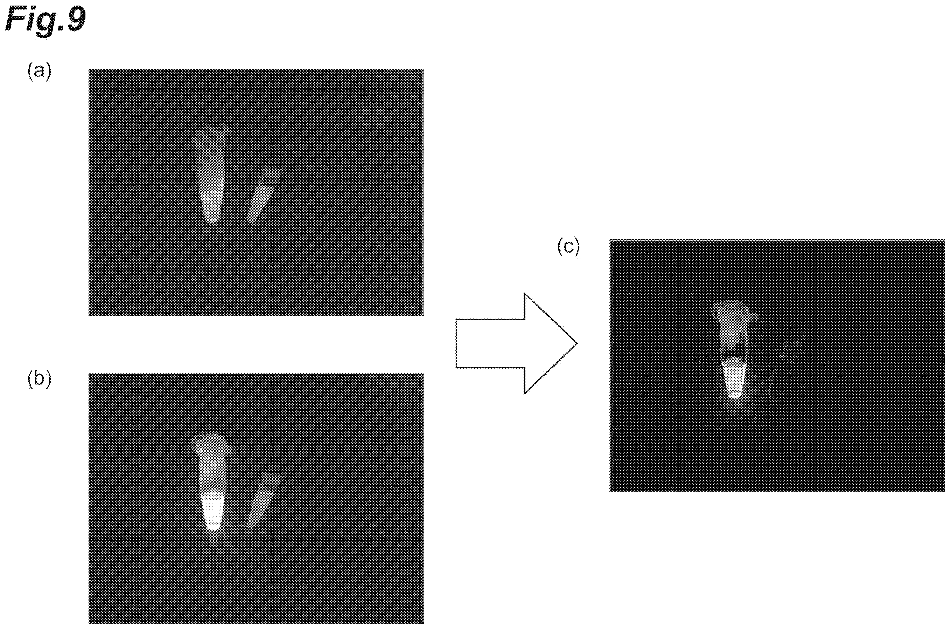

Next, an experimental result of generation of an analysis image in the simulation device 100 simulating the living body observation device 1A of the above embodiment is shown. FIG. 7 illustrates a configuration of an experimental system including the simulation device 100, FIG. 8 illustrates an image indicating a disposition state of the sample that is an experiment target, and FIG. 9 illustrates an analysis image of the experimental result.

As illustrated in FIG. 7, a device in which the excitation light source 105 and the dichroic mirror 109 were attached to a smartphone 115 was prepared as the simulation device 100, and the simulation device 100 was disposed to face two samples S1 and S2 taken as the specimen S. As illustrated in FIG. 8, the samples S1 and S2 were placed side by side, and each of the samples S1 and S2 was a sample assumed to be cancer cells and adipose tissue. Specifically, the samples S1 and S2 were prepared as samples in which a fluorescent dye having a concentration of several nmol was placed in a transparent container. Fluorescein (FITC) having a fluorescence wavelength of 525 nm and an excitation wavelength of 470 nm was used as the fluorescent dye. Therefore, a dichroic mirror 109 having characteristics of transmitting light having a fluorescence wavelength of 525 nm and reflecting excitation light including an excitation wavelength of 470 nm was attached to the smartphone 115. Further, a shutter speed of the camera was set to 1/15 sec using the smartphone 115.

FIG. 9(a) illustrates a fluorescence image of the samples S1 and S2 acquired by the simulation device 100, and this image simulates the reference image. Further, FIG. 9(b) illustrates a fluorescence image of the samples S1 and S2 acquired by the simulation device 100 in a state in which a concentration of the fluorescent dye in the sample S1 is increased as compared with a case in which the fluorescence image of FIG. 9(a) has been obtained, and this image simulates the target image. That is, in the fluorescence image illustrated in FIG. 9(b), a fluorescent image after a predetermined time has elapsed after the fluorescent reagent is sprayed is simulated. Further, FIG. 9(c) illustrates a difference image generated on the basis of the fluorescence image illustrated in FIG. 9(a) and the fluorescence image illustrated in FIG. 9(b), and this difference image simulates the analysis image. From this result, it can be seen that the fluorescence image of only the sample S1 hypothesized as cancer cells appears in the analysis image acquired by the simulation device 100. From this fact, it was verified that the determination of the presence or absence and the position of cancer cells by the living body observation devices 1, 1A, and IB functioned adequately.

Here, the observation target of the living body observation device of the above embodiment is a target to which a fluorescent substance is applied. The fluorescent substance is a substance that emits fluorescence by reacting with cancer cells and has an excitation wavelength in a range of 300 nm to 650 nm. In this case, it is possible to improve the accuracy of the determination of the presence or absence or the position of cancer cells present on the surface of the observation target. Further, the observation light of the living body observation device of the above embodiment may include autofluorescence having an excitation wavelength in the range of 300 nm to 450 nm.

In the generating of the analysis image, it is preferable to generate a difference between the reference image and the target image as an analysis image. It is preferable for the image processing device to generate a difference between the reference image and the target image as the analysis image. In this case, it is possible to clearly visualize the portion in which change in the light intensity of the target image with respect to the reference image is large. Accordingly, it is possible to improve the accuracy of determination of the presence or absence or the position of cancer cells in the tissue.

Further, in the storing of the reference image, it is preferable for the image of the observation light immediately after the fluorescent substance is applied to be stored as the reference image. Further, it is preferable for the storage unit to store the image of the observation light immediately after the fluorescent substance is applied as the reference image. In this case, it is possible to easily acquire change in the light intensity of the target image with respect to the reference image. As a result, it is possible to ensure accuracy in determination of the presence or absence or the position of cancer cells in the tissue.

Further, in the storing of the reference image, it is also preferable to store the image of the observation light after a predetermined time has elapsed after the fluorescent substance is applied as a reference image. It is also preferable for the storage unit to store the image of the observation light after a predetermined time has elapsed after the fluorescent substance is applied as the reference image. In this case, it is possible to easily acquire change in the light intensity of the target image with respect to the reference image. As a result, it is possible to ensure accuracy in determination of the presence or absence or the position of cancer cells in the tissue.

Further, in the generating of the analysis image, it is also preferable for the image of the observation light after at least three minutes have elapsed after the fluorescent substance is applied to be acquired as the target image. Further, it is also preferable for the image processing device to acquire the image of the observation light after at least three minutes have elapsed after the fluorescent substance is applied as the target image. In this case, it is possible to clearly visualize change in the light intensity of the target image with respect to the reference image. As a result, it is possible to improve the accuracy of the determination of the presence or absence or the position of cancer cells in the tissue.

Further, in the living body observation method of the above embodiment, it is preferable for the camera and the image processing device to be built into the smart device. Furthermore, in the living body observation device of the above embodiment, it is preferable for the imaging unit and the image processing device to be built into the smart device. With such a configuration, it is possible to realize the living body observation method or the living body observation device of the above-described embodiment with a simple device configuration.

Further, in the living body observation method according to the above embodiment, it is also preferable for the light source to be electrically connected to the smart device, and for the smart device to be configured to control the light source so that the light source radiates the excitation light at the capturing timing of the camera. Further, in the living body observation device of the above embodiment, it is also preferable for the light source to be electrically connected to the smart device, and for the smart device to be configured to control the light source so that the light source radiates the excitation light at the capturing timing of the imaging unit. With such a configuration, it is possible to efficiently generate an analysis image indicating change in the light intensity of the target image with respect to the reference image.

Further, in the living body observation method of the above embodiment, it is preferable for the light source to be built into the smart device, and for the smart device to be configured to control the light source so that the light source radiates the excitation light at the capturing timing of the camera. Further, in the living body observation device of the above-described embodiment, it is preferable for the light source to be built into the smart device, and for the smart device to be configured to control the light source so that the light source radiates the excitation light at the capturing timing of the imaging unit. With such a configuration, it is possible to efficiently generate an analysis image indicating change in the light intensity of the target image with respect to the reference image.

### Industrial Applicability

According to one aspect of the present invention, it is possible to accurately determine the presence or absence or the position of cancer cells on the surface of a tissue using the living body observation method and the living body observation device for observing an observation target.

### Reference Signs List

1, 1A, 1B, 1C Living body observation device
5, 5C Excitation light source
11 Excitation light shielding filter
13, 13A, 13C Camera (imaging unit)
15 Computer
15A, 15C Smart device
21a Control unit
21b Image processing unit (image processing device)
21c Data storage unit
23 Display (output device)
S Specimen (observation target)

## Claims

1. A living body observation method for observing a surface of an observation target excised from a living body, the method comprising:
applying a fluorescent substance that emits fluorescence by binding to a cancer cell and has an excitation wavelength in a range of 300 nm to 650 nm to the observation target;
irradiating the observation target with excitation light including the excitation wavelength using a light source;
cutting the excitation light from observation light emitted from the observation target using a filter;
capturing the observation light from which the excitation light is cut and outputting image data using a camera;
storing a reference image which is an image of the observation light after the fluorescent substance is applied on the basis of the image data;
acquiring a target image which is an image of the observation light after acquisition of the reference image on the basis of the image data and generating an analysis image indicating change in light intensity of the target image with respect to the reference image using an image processing device; and
outputting the analysis image using an output device.

2. The living body observation method according to claim 1, wherein the generating of the analysis image includes generating a difference between the reference image and the target image as the analysis image.

3. The living body observation method according to claim 1 or 2, wherein the storing of the reference image includes storing an image of the observation light immediately after the fluorescent substance is applied as the reference image.

4. The living body observation method according to claim 1 or 2, wherein the storing of the reference image includes storing an image of the observation light after a predetermined time has elapsed after the fluorescent substance is applied as the reference image.

5. The living body observation method according to any one of claims 1 to 4, wherein the generating of the analysis image includes acquiring an image of the observation light after at least three minutes have elapsed after the fluorescent substance is applied as the target image.

6. The living body observation method according to any one of claims 1 to 5, wherein the camera and the image processing device are built into a smart device.

7. The living body observation method according to claim 6, wherein:
the light source is electrically connected to the smart device, and
the smart device is configured to control the light source so that the light source radiates the excitation light at an capturing timing of the camera.

8. The living body observation method according to claim 6,
wherein the light source is built into the smart device, and
the smart device is configured to control the light source so that the light source radiates the excitation light at an capturing timing of the camera.

9. A living body observation device that allows observation of a surface of an observation target, the living body observation device comprising:
a light source configured to radiate excitation light including a wavelength between 300 nm and 650 nm;
a filter configured to cut the excitation light in observation light emitted from the observation target irradiated with the excitation light;
an imaging unit configured to capture the observation light from which the excitation light is cut, and output image data;
a storage unit configured to store a reference image that is an image of the observation light emitted from the observation target on the basis of the image data;
an image processing device configured to acquire a target image that is an image of the observation light after acquisition of the reference image on the basis of the image data and generate an analysis image indicating change in light intensity of the target image with respect to the reference image; and
an output device configured to output the analysis image.

10. The living body observation device according to claim 9, wherein the observation target is a target to which a fluorescent substance is applied, the fluorescent substance being a substance that emits fluorescence by reacting with cancer cells and having an excitation wavelength in a range of 300 nm to 650 nm.

11. The living body observation device according to claim 10, wherein the image processing device generates, as the analysis image, a difference between the reference image and the target image.

12. The living body observation device according to claim 10 or 11, wherein the storage unit stores, as the reference image, an image of the observation light immediately after the fluorescent substance is applied.

13. The living body observation device according to claim 10 or 11, wherein the storage unit stores, as the reference image, an image of the observation light after a predetermined time has elapsed after the fluorescent substance is applied.

14. The living body observation device according to any one of claims 10 to 13, wherein the image processing device acquires, as the target image, an image of the observation light after at least three minutes has elapsed after the fluorescent substance is applied.

15. The living body observation device according to claim 9, wherein the observation light includes autofluorescence having an excitation wavelength in a range of 300 nm to 450 nm.

16. The living body observation device according to any one of claims 9 to 15, wherein the imaging unit and the image processing device are built into a smart device.

17. The living body observation device according to claim 16, wherein:
the light source is electrically connected to the smart device, and
the smart device is configured to control the light source so that the light source radiates the excitation light at an capturing timing of the imaging unit.

18. The living body observation device according to claim 16,
wherein the light source is built into the smart device, and
the smart device is configured to control the light source so that the light source radiates the excitation light at an capturing timing of the imaging unit.
